# EUROPEAN PATENT SPECIFICATION

(11) **EP 2 950 739 B1**
(45) Date of publication and mention of the grant of the patent: **16.06.2021**
(21) Application number: 14706997.5
(22) Date of filing: 30.01.2014
(51) Int. Cl.: A61B 42/00

(54) **ERGONOMIC GLOVE FOR MEDICAL PROCEDURES**
ERGONOMISCHER HANDSCHUH FÜR MEDIZINISCHE EINGRIFFE
GANT ERGONOMIQUE POUR PROCÉDURES MÉDICALES

(30) Priority: 30.01.2013 US 201361758728 P; 04.03.2013 US 201361772463 P; 18.12.2013 US 201314133438
(43) Date of publication of application: 09.12.2015
(73) Proprietor: Exsomed International IP, LLC, Avarua, Rarotonga (CK)
(72) Inventor: CHAMPAGNE, Lloyd P., Phoenix, AZ 85004 (US); ZOLDOS, Jozef, Phoenix, AZ 85004 (US); DEROS, Yani, Phoenix, AZ 85004 (US)
(74) Representative: Pallini Gervasi, Diego
(86) International application number: PCT/US2014/013940
(87) International publication number: WO 2014/120972

(56) References cited:
- WO-A1-96/39055
- WO-A1-2013/126727
- FR-A- 1 141 139
- US-A- 5 644 797
- US-A1- 2002 166 156
- US-A1- 2006 005 295

## Description

### FIELD OF THE INVENTION

The present invention relates to a surgical glove formed to be all or partially in essentially the relaxed shape of a human hand, potentially including being formed in a flexed position at one or more of the finger joints, palm and/or dorsum of the hand. The invention also relates to specific features on the glove to alleviate biasing forces related to movement of the hand and to assist the glove in conforming to the movement of the hand.

### BACKGROUND OF THE INVENTION

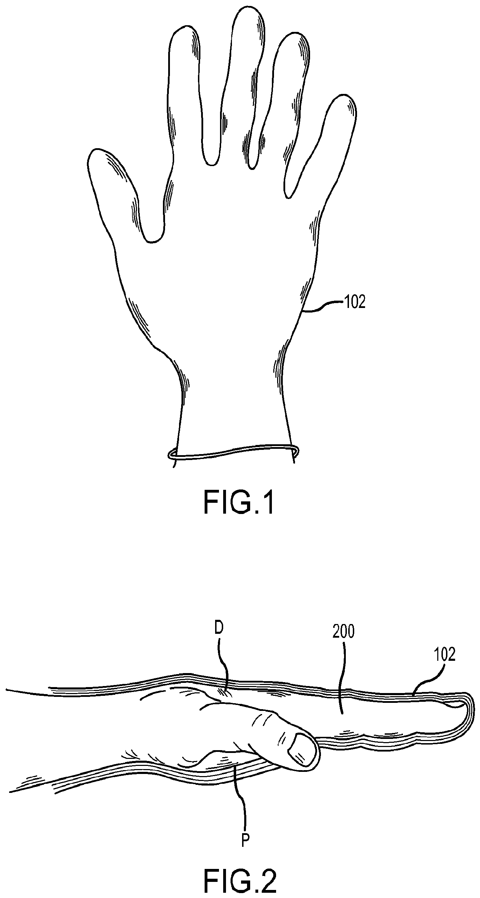

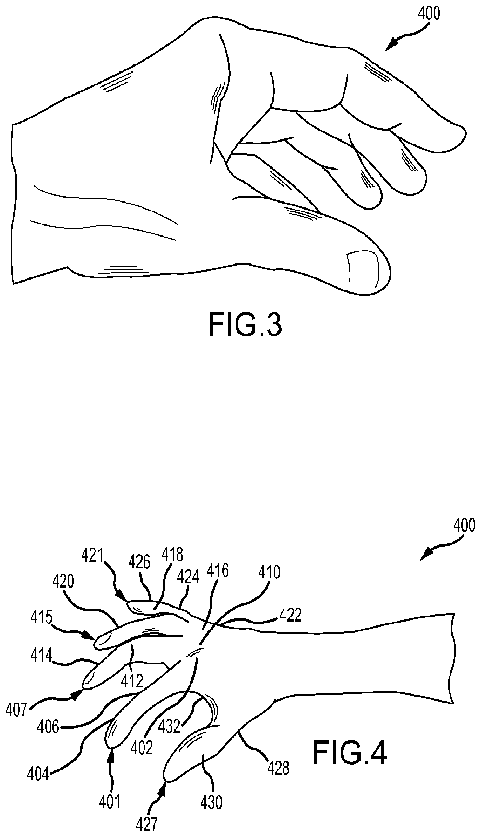

Surgical gloves are known in the art. The gloves are usually manufactured from latex or latex-free substitutes and fit snugly on the hand. Surgical gloves are often initially formed in a shape approximately the same as a hand when pressed flat on a surface or extended to be essentially flat or straight, such as shown in FIG. 1 and FIG. 2. In that position, the fingers extend outward, essentially straight from the palm (in this context "straight" means there is essentially no bend at any of the joints). In this position, the thumb is oriented in a flat plane or is slightly abducted away from the palm. A problem with the standard glove shape is that the relaxed hand is not naturally in a flat position with the fingers essentially straight. As shown in FIGS. 3-4, when in its normal, relaxed position, which is also called the normal hand cascade position, the joints of the fingers (the fingers and thumb also collectively referred to herein as "digits") are naturally in a flexed position, with the thumb in a different plane than the fingers. This normal position does not match the shape of a standard surgical glove.

If a medical professional wears surgical gloves for a long period, such as when he/she is performing a long procedure or is performing multiple successive procedures in a given period of time, the professional's fingers and hands can become tired or fatigued because of constantly overcoming the biasing forces of the surgical glove(s) in order to flex the fingers and hand (either to a closed position, open position, or both).

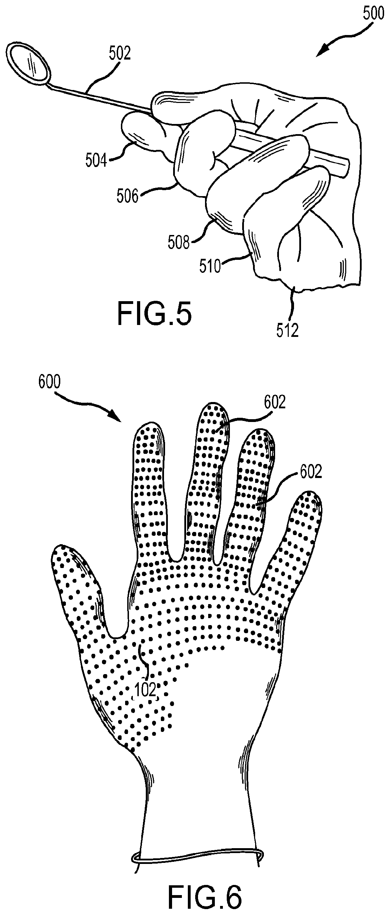

Consequently, when a standard surgical glove is placed on a hand, the material of the glove biases the fingers away from the normal, relaxed position to the less natural straight position. When a medical professional then uses his/her hand during a medical procedure, in order to flex the fingers, the biasing force of the glove material must be overcome. For example, FIG. 5 shows a hand 500 of a medical professional grasping a dental instrument 502. Fingers 504-510 are flexed to grasp the instrument 502, and to do so, the biasing force of surgical glove 512 must be overcome. This biasing force is even greater if two gloves are placed on the hand, which is frequently done to increase protection for the medical professional in the event that the outer glove tears or is punctured or if there is a random manufacturing defect resulting in a perforation.

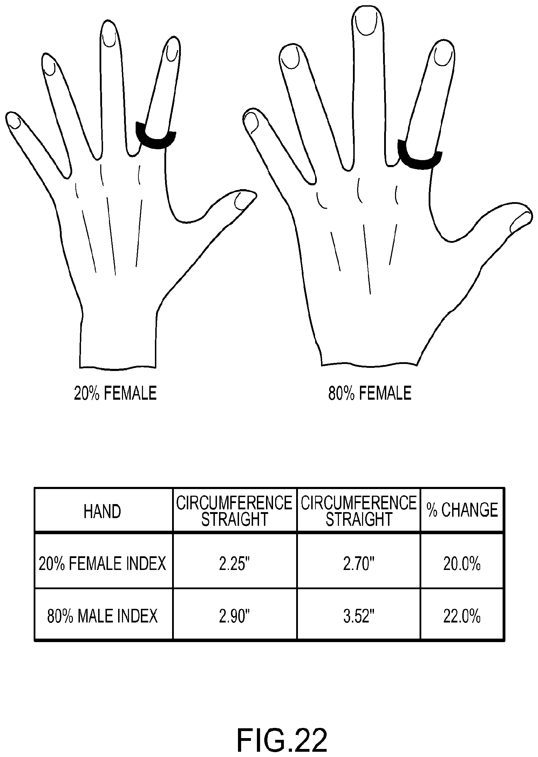

In addition to standard surgical gloves being formed in a straight position, they have no structure to permit the expansion or contraction of the dimensions of portions of the hand. For example, the circumference of a flexed finger (such as when the fingers are flexed towards the palm of the hand) is greater than its circumference when relaxed or in the straight position. This concept is illustrated in FIG. 22, showing a 20% increase in circumference in a female index finger and a 22% increase in circumference in a male index finger. If gloves are designed so they tightly fit fingers that are in the straight position, and then the fingers are flexed, the fingers must also overcome the biasing force of the glove material that restricts digital expansion. Consequently, there is a need for extra material during flexion of the fingers so the portion of the glove covering the portion of the finger that expands can (1) permit expansion when the finger is flexed, and (2) contract back into shape and is not used when the finger is not flexed. The biasing force of gloves also includes adduction of the fingers, a force tending to keep the fingers together in line rather than in their natural cascading position. This is another biasing force that must be overcome when using standard surgical gloves.

Glove designs with baggy, or loose-fitting portions, at one or more areas are known, but such gloves are not optimal for a medical professional performing procedures that require fine, precise work. Glove designs are also known that have ribs at some areas, but while the ribs may help to some degree, they do not overcome the problems described herein.

The US Patent document No 5,644,797 discloses a puncture resistant glove is shown which includes a puncture resistant layer disposed on non-puncture resistant portions for protection of a wearer's digits. The puncture resistant layer includes aseries of shields of puncture resistant material which overlap at the joints to protect the joint, but allow flexing of the joint. The glove may also include accordion-shaped zones which allow easier flexing and extension of the glove.

The US Patent application publication No US 2002/166156 A1 discloses a flexible and stretchable glove made of elastomeric material, the glove having palm, back, and sleeve elements with thumb and finger portions. A plurality of corrugations at high flex points substantially parallel to the direction of the axis of flexure reduce the stress on the hand induced by tension in the elastomeric material when stretched by the bending motions of the hand, thumb and fingers.

The French Patent Nr. Fr 1141139 A discloses an anatomical glove made of rubber or the like, essentially comprising, in at least one zone of finger joints, a certain clearance intended to facilitate the folding of the fingers and the hand.

The international Patent application publication No. WO 96/39055 A1 discloses surgical gloves that may be used for either or both of the dominant and non-dominant hands of a surgeon. Other users may use the disclosed gloves individually or as a pair to perform other applicable functions. The described glove should fit snugly and with comfort. Specifically, this publication discloses gloves including puncture resistant material in one solid sheet (106) at a thickness less than, equal to, or greater than those previously utilized. A glove is disclosed with a thickness greater than those previously designed in areas not critical in tactile sensation, which in turn provides greater puncture protection than those previously designed because it is a solid film which is impervious to body fluids and does not need additional films to act as the fluid resistant barrier. The disclosed gloves can be of greater thickness in areas not concerned with tactile sensation due to the flex point and/or wedge design.

The international Patent application publication No. WO 2013/126727 A (document falling under Article 54(3) EPC) discloses a glove having both anatomically correlating stress relief zones and/or reinforced zones to add support in areas opposite the stress relief zones to keep bunching or slipping of the glove from interfering with the bending of the user's joints. The described glove may have corrugated relief zones over some or all of the joints and knuckles of the hand, wherein the relief zones are formed of peaks and valleys. A relief zone can also be provided over the webbing between the thumb and the palm.

It would be beneficial for medical professionals to have surgical gloves that minimize biasing forces, that include a minimal amount of excess, loose or baggy material, and that are relatively simple to manufacture, so they are cost effective.

### SUMMARY OF THE INVENTION

In accordance with the present invention, there is provided a surgical glove according to claim 1. Further embodiments of the invention are provided by the dependent claims.

According to the present invention, a glove as described herein includes longitudinal ribs along parts of one or more fingers or the thumb to allow for expansion of the digit (i.e., an increase in circumference during flexing). Furthermore, a pattern can exist in one or more spaces between the fingers, or the thumb and index finger, or the thumb and index finger, thus reducing the biasing force of the glove in that area(s) and allowing easier abduction or movement. Also, if the glove is formed with flex angles, it may have a bias toward flexion, rather than extension (the opening of the hand), so features can be added on the palm side of the glove, such as at the thumb, and/or one or more finger creases and/or center of the palm to relieve some of the force of opening or closing the hand and/or digits.

Further, exemplary gloves can include patterns and/or shapes at any suitable location, including at the joint portions including the flex angles. These reduce the biasing force to close and/or open the hand.

In accordance with further exemplary embodiments, a glove may include material of various thicknesses. For example, a glove can include material of a first thickness corresponding to most of the hand, and have material that is thinner at the portions of the hand (which includes the digits) that flex when opening or closing, e.g., at one or more joints of the digits and/or on the dorsum or palm.

In accordance with yet further exemplary embodiments, a glove includes material in some areas that is more flexible in areas where the digits and hand flex, and may include another material that is more puncture resistant than the glove material in other areas. For example, the glove can include reinforced material on the fingertips and/or palm areas and a second material that flexes more at one or more of the joints and/or the dorsum or palm.

Gloves in accordance with the present disclosure can also include any combination of materials, flex angles, patterns, and features as described herein.

As set forth in more detail below, various gloves as described herein can accommodate the movement of the hand and digits with reduced biasing force, and at the same time fit properly (and preferably not be oversized or baggy) throughout the hand's entire range of motion, especially the range used during a medical procedure.

### BRIEF DESCRIPTION OF THE DRAWINGS

FIG. 1 depicts a hand that is flat with the fingers extending outward from the palm in essentially a straight position, with a surgical glove not in accordance with the present invention on the hand.
FIG. 2 depicts a partial cross-sectional view of the surgical glove of FIG. 1.
FIG. 3 depicts a hand in a suspended, normal relaxed position and not resting on a surface.
FIG. 4 illustrates a hand in a normal, relaxed position.
FIG. 5 depicts a hand with a conventional surgical glove not according to the present invention thereon, wherein the hand is grasping a dental instrument.
FIG. 6 depicts a top view (wherein the flexed angles of the joints cannot be readily seen) of an exemplary glove that includes a pattern (not according to the present invention).
FIG. 6A is a close-up, side illustration of an exemplary pattern suitable for the material of the glove of FIG. 6 (not according to the present invention).
FIG. 6B is a close-up, side illustration of another exemplary pattern suitable for the material of the glove of FIG. 6 (not according to the present invention).
FIG. 6C is a close-up, side illustration of another exemplary pattern suitable for the material of the glove of FIG. 6 (not according to the present invention).
FIG. 6D is a close-up, side illustration of another exemplary pattern suitable for the material of the glove of FIG. 6 (not according to the present invention).
FIG. 6E is a close-up, side illustration of another pattern suitable for the material of the glove of FIG. 6 (not according to the present invention).
FIG. 7 depicts a top view (wherein the flex angles of the joints cannot be readily seen) of an exemplary glove (not according to the present invention) that includes a pattern of ribs.
FIG. 8 depicts a top view (wherein the flex angles of the joints cannot be readily seen) of an exemplary glove (not according to the present invention) that includes a pattern of ribs in selected portions of the glove.
FIG. 9 depicts a top view (wherein the flex angles of the joints cannot be readily seen) of an exemplary glove (not according to the present invention) that includes a pattern of ribs in selected portions of the glove, wherein the ribs are axially oriented to the digits.
FIG. 10 depicts a top, perspective view of an exemplary glove (not according to the present invention) that includes a pattern of ribs in selected portions of the glove.
FIG. 11 depicts a top, perspective view of a glove in accordance with an alternate embodiment not according to the present invention.
FIG. 12 illustrates a hand with a standard surgical glove not according to the present invention wherein the hand is biased to flexion.
FIG. 13A illustrates a palm view of a standard surgical glove (not according to the present invention).
FIG. 13B illustrates a side view of the glove (not according to the present invention) of FIG. 13A.
FIG. 14 illustrates comparative strain forces of fingers moving from the straight to the flexed position for various gloves.
FIG. 15 illustrate another glove having a pattern in accordance with additional embodiments not according to the present invention.
FIGS. 16(a) and 16(b) illustrate another glove having a pattern not in accordance with the present invention.
FIG. 17 illustrates a hand without a surgical glove, wherein the hand is fully biased to the flexion position.
FIG. 18A illustrates a side view of a hand in the straight position, which is the position in which most current surgical gloves are formed.
FIG. 18B illustrates a palm view of the hand of FIG. 18A.
FIG. 19A illustrates a side, perspective view of a hand in a fully relaxed position.
FIG. 19B illustrates a front view of the hand in FIG. 19A.
FIG. 20 illustrates a hand in a working position.
FIG. 21 illustrates a hand in a more natural position, but not the fully relaxed position, and shows the angles of the finger joints when in this position.
FIG. 22 illustrates a relationship between the circumference of fingers when straight and when flexed.
FIG. 23 illustrates an amount of stretch and location of the stretch for a size 6.5 glove at the MCP joints of the fingers.
FIG. 24 illustrates an amount of stretch and location of the stretch for a size 8 glove at the MCP joints of the fingers.
FIG. 25A illustrates the hand in a flat position with the figures extended.
FIG. 25B illustrates the hand of FIG. 25A with the fingers flexed to essentially a fully-closed position.
FIG. 26 is a top view of a hand illustrating the amount of extra material or stretch (as measured from the MCP joints) for the fingers to move from a straight to a cascading position for a size 8 glove.
FIG. 27 (not according to the present invention) illustrates the amount of extra material or stretch (as measured from the MCP joints) for the fingers to move from a straight to a cascading position for a size 6.5 glove.
FIG. 28A is a palm view of a glove in accordance with an embodiment of the invention.
FIG. 28B is a side view of the glove of FIG. 28A.
FIG. 28C is a top view of the glove of FIG. 28A.
FIG. 29 is a top view of a hand.

### DETAILED DESCRIPTION OF PREFERRED EMBODIMENTS

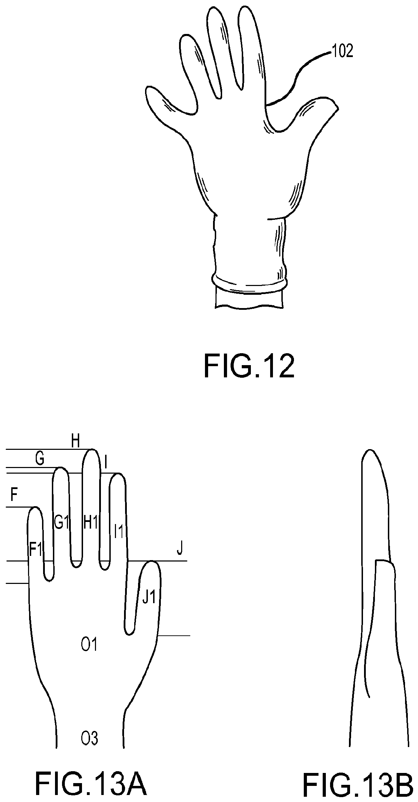

Turning now to the drawings where the purpose is to describe exemplary embodiments of the invention and not to limit the same, FIG. 1 illustrates a top view, FIG. 2 illustrates a side view, and FIG. 12 illustrates a bottom or palm view of a hand 200 in a relatively flat position with a surgical glove 102 thereon. A shape of a conventional glove is further illustrated in FIGS. 13(a) and 13(b), which illustrate a palm view and a side view of the conventional glove.

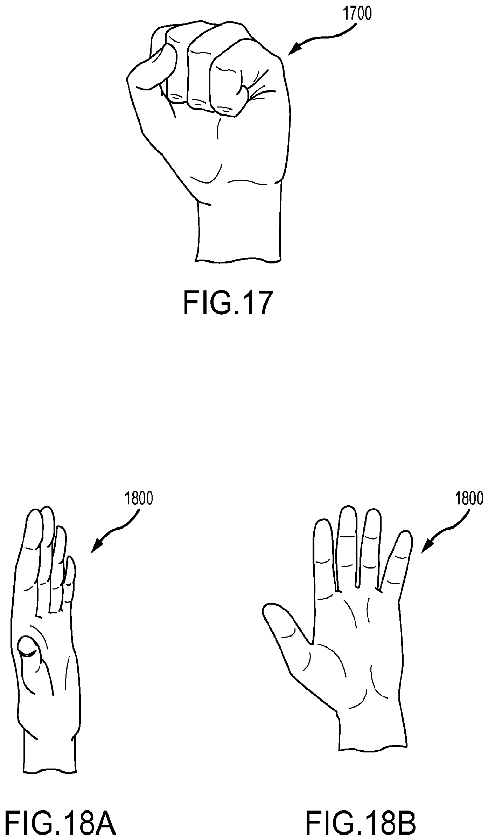

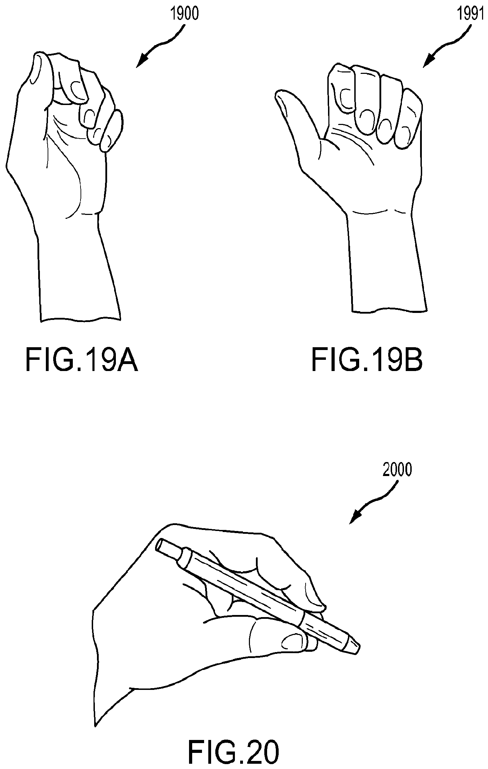

To facilitate understanding of the gloves described herein, various hand positions are illustrated in FIGS. 17-21. FIG. 17 illustrates a hand 1700 biased to flexion. FIG. 18A illustrates a side view of a hand 1800 in the straight position, which is the position in which standard surgical gloves are formed. FIG. 18B illustrates a palm view of hand 1800. FIG. 19A illustrates a side, perspective view of a hand 1900 in a fully relaxed position and having cascading fingers. FIG. 19B illustrates a front view of the hand 1900. FIG. 20 illustrates a hand 2000 in a working position.

As is known, an MCP joint is a metacarpophalangeal joint. A PIP joint is a proximal interphalangeal joint. A DIP joint is a distal interphalangeal joint. An IP joint is an interphalangeal joint. And, a CMC joint is a carpometacarpal joint.

A thumb generally includes three joints. The most proximal thumb joint is the CMC joint between the trapezium and the thumb metacarpal. The thumb MCP joint is between the metacarpal and the proximal phalanx of the thumb. The distal most thumb joint is the IP or interphalangeal between the proximal and distal phalanges of the thumb.

Each finger, including the index, middle, ring and little fingers, has four joints including the CMC, MCP, PIP and DIP joints. The CMC joint of each finger is between the metacarpal and the carpal bone. The MCP joint of each finger is between the metacarpal and the proximal phalanx. The PIP joint of each finger is between the proximal and middle phalanges. The DIP joint of each finger is between the middle and distal phalanges.

Turning to Figure 4, respective portions of a hand 400 are shown. Index finger 401 has MCP joint 402, DIP joint 404, and PIP joint 406. Middle finger 407 has MCP joint 410, PIP joint 412, and DIP joint 414. Ring finger 415 has MCP joint 416, PIP joint 418 and DIP joint 420. Little finger 421 has MCP joint 422, PIP joint 424 and DIP joint 426. Thumb 427 has MCP joint 428 and IP joint 430. There is a space 432 between the thumb 427 and index finger 401. Not readily visible are the CMC joints of the fingers and thumb.

Surgical gloves as described herein can be formed of any suitable material, such as medical-grade natural rubber latex or synthetic rubber material. Exemplary synthetic rubber materials include polychloroprene (neoprene), polyisoprene, styrene butadiene, styrene ethylene butadiene. Other suitable materials include nitrile and vinyl (polyvinylchloride). The thickness of the glove material may be any suitable amount and can range between, as an example, 0.14mm - 0.3mm, and surgical glove thicknesses are known in the art. Surgical gloves come in different sizes, with typical sizes ranging from 5½ to 9, which is also known in the art.

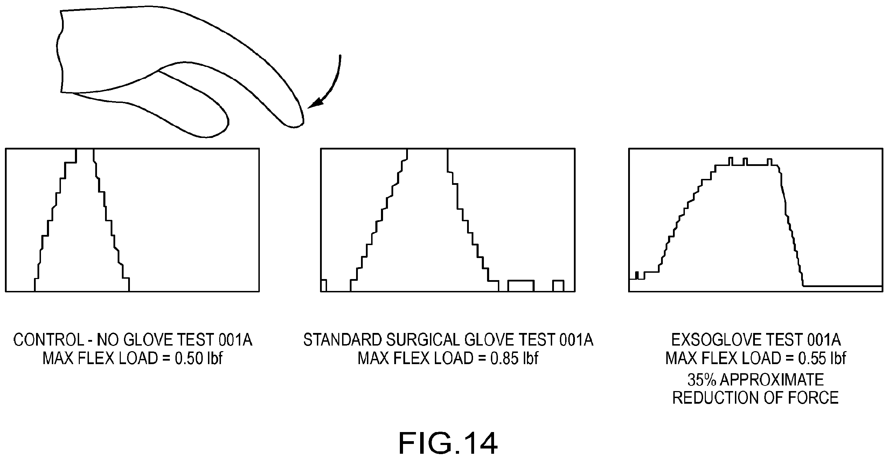

Various embodiments of the glove designs described herein can be used alone or in any combination with the other designs and features noted herein. As noted herein, various advantages of the gloves herein described include a reduction of a biasing forces when the hand is opened and/or closed. FIG. 14 illustrates an exemplary reduction in flex load, compared to standard gloves. In this specific illustrated example, gloves in accordance with the present description reduced a flex load by about 35%, although the invention is not limited to this amount.

When referring to a location on a glove according to the invention, it may simply be referenced by the position on the hand (e.g., MCP joint of the index finger) or as a "location," "portion," or "position" or "location of the glove," "portion of the glove," or "position of the glove."

### Flex Angles

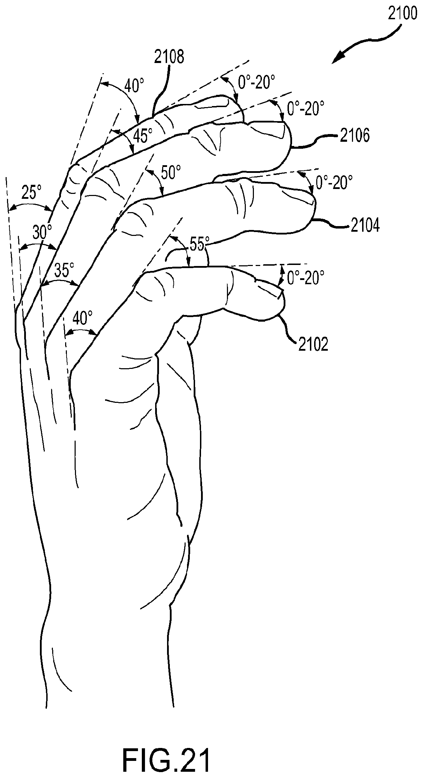

Exemplary gloves according to various embodiments of the invention are formed so the portions of the gloves corresponding to the fingers and/or thumb of a hand are more generally in a normal, relaxed hand position, but not entirely in the relaxed position, such as the position illustrated in Figure 21. In this manner the hand can be closed and opened with limited resisting biasing forces on one or more of the digits and/or hand caused by the glove. By way of examples, portions of a glove corresponding to one or more of the MCP joints, one or more of the PIP joints and/or one or more of the DIP joints of a hand can be formed at a flex angle. A surgical glove according to exemplary embodiments the invention preferably includes one or more of the following portions formed as follows: (a) the portion corresponding to the MCP joint of the index finger is formed at a flex angle of 10-45°, or 40°-50° (b) the portion corresponding to the MCP joint of the middle finger is formed at a flex angle of 10-45°, or 40°-50° (c) the portion corresponding to the MCP joint of the ring finger is formed at a flex angle of 15-50°, or 40°-50°, and (d) the portion corresponding to the MCP of the little finger formed at a flex angle of 20-55°, or 40°-50°.

Further, the following portions of the glove may be formed as follows: (a) the portion corresponding to the PIP joint of the index finger is formed at a 5-45°, or 10°-20°, flex angle, (b) the portion corresponding to the PIP joint of the middle finger is formed at a 5-50° or 10°-20°, flex angle, (c) the portion corresponding to the PIP joint of the ring finger is formed at a 10-55° or 10°-20°, flex angle, and (d) the portion corresponding to the PIP joint of the little finger is formed at a 15-60°, or 10°-20°, flex angle.

Also, (a) the DIP joint of the index finger may be formed at a 5°-25°, or 10°-20°, or 0°-20°, flex angle, (b) the DIP joint of the middle finger may be formed at a 5°-25°, or 10°-20°, or 0°-20°, flex angle, (c) the DIP joint of the ring finger may be formed at a 10°-30°, or 10°-20°, or 0°-20°, flex angle, and (d) the DIP joint of the little finger is formed at a 15°-30°, or 10°-20°, or 0°-20°, flex angle.

Additionally or alternatively, one or more of the following portions of a glove corresponding to the thumb may be formed as follows: (a) the portion corresponding to the MCP joint of the thumb is formed at a flexed angle of 10-45°, and (b) the portion corresponding to the IP joint of the thumb is formed at a flexed angle of 20-50°. Other flex angles are disclosed in the exemplary preferred embodiments included herein.

Additionally, gloves according to various exemplary embodiments of the invention can include a two-radius curve, meaning that the angles of the MCP joints are flexed at a greater angle than the other joints.

In one embodiment, the portions of the glove corresponding to the MCP joints have greater flex angles than either the PIP and DIP joints, such that the fingers in the hand remain open to some degree, rather than being in their fully relaxed position. One embodiment of preferred flex angles are approximately:

| | Index finger | Middle finger | Ring finger | Little finger |
|---|---|---|---|---|
| MCP joint | 25° | 25° | 30° | 35° |
| PIP joint | 15° | 15° | 20° | 25° |
| DIP joint | 15° | 15° | 20° | 25° |

Although these angles are less than the complete relaxed position of the hand, they allow for: ease of manufacturing, helping to keep the hand open to some degree, and preventing too much bias against opening or closing the hand.

Any of the flex angles set forth herein could possibly be varied beyond the stated ranges to better approximate the shape of a hand in a position to reduce biasing forces when the hand opens or closes.

Another aspect of the invention is that when in a relaxed position, the fingers may be in a cascading position moving from the index finger to the little finger, with the flex angles of the joints of each finger varying. An example of a hand 2100 including cascading fingers 2102-2108 is illustrated in Figure 21. Some embodiments of the gloves disclosed herein include this cascading feature. One way in which a glove according to the invention compensates for this cascading position is by providing different flex angles, or features to permit flexing, at the various joints. Exemplary angles for the cascading digits are illustrated in Figure 21, and these angles may fall within ranges as set forth in the exemplary embodiments herein.

Additionally, the palm region of the glove may be formed at a suitable flex angle, such as between 10°-30°. Moreover, a glove according to the invention may have the thumb portion formed in a more natural position with the thumb in a different plane than the fingers.

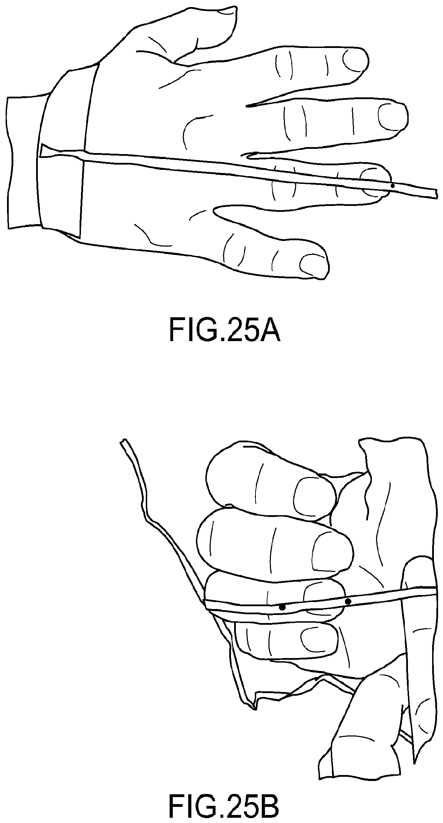

When the fingers are flexed from an extend to a closed position, as illustrated in Figures 25A-25B, the length along the top of the finger, measured from a position just behind the MCP joint of the finger increases. For a size 6.5 glove and 8.0 glove, the length along the top of each finger increases by approximately the amounts shown below:

| | Index finger | Middle finger | Ring finger | Little finger |
|---|---|---|---|---|
| Size 6.5 glove | 10mm | 13mm | 20mm | 32mm |
| Size 8.0 glove | 11mm | 15mm | 24mm | 30mm |

The difference in increased length moving from the index finger to the little finger is because of the cascading shape of the hand when relaxed or closed.

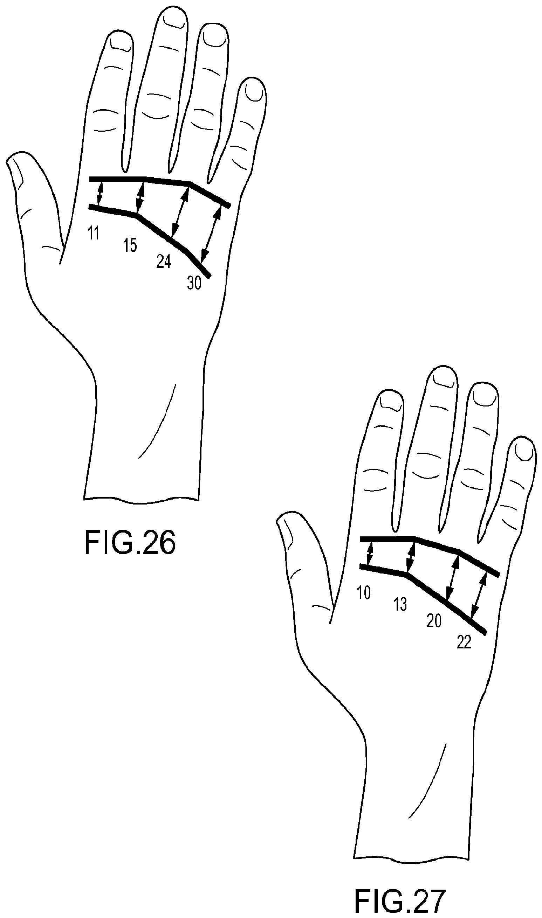

Therefore, it is desirable to provide extra material for the tops of the fingers (and thumb) when in the closed position to reduce biasing forces. This can be done by pre-flexing the glove as described herein so it is formed in a position that is close to, but preferably not quite, in the position of a relaxed hand and/or by including patterns. In one embodiment corresponding to the above chart, all of the extra material is provided at the MCP joint of each finger, as illustrated in Figure 26 (for a size 8.0 glove) and 27 (for a size 6.5 glove). The extra material is preferably (a) half provided by the flex angles for the MCP joints disclosed herein, and (b) half provided by patterns (discussed below). Alternatively, the extra material may be entirely provided by flex angles at two or more joints of a finger, or by flex angles at two or more joints of each finger and patterns at one or more joints of each finger.

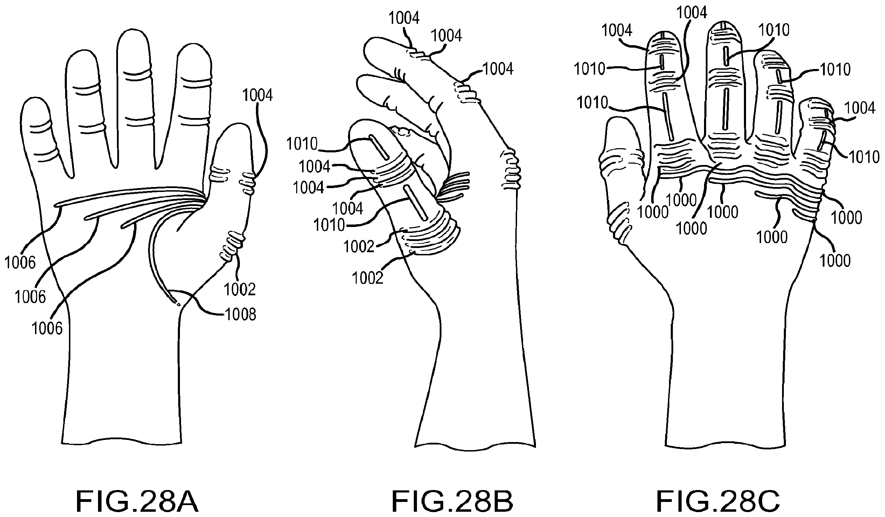

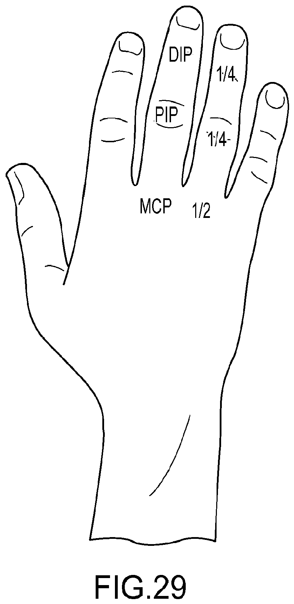

Figures 28A-28C show a glove in accordance with the invention wherein approximately 1/2 of the extra material required along the top side of the finger when fully flexed is provided by flex angles at each finger joint and about half is provided by patterns, which are preferably ribs at each joint. As shown in Figure 29, in one embodiment, about 1/2 of the extra length required for fully closing each finger is provided at the MCP joint, about 1/4 is provided at the PIP joint and about 1/4 is provided at the DIP joint.

### Patterns

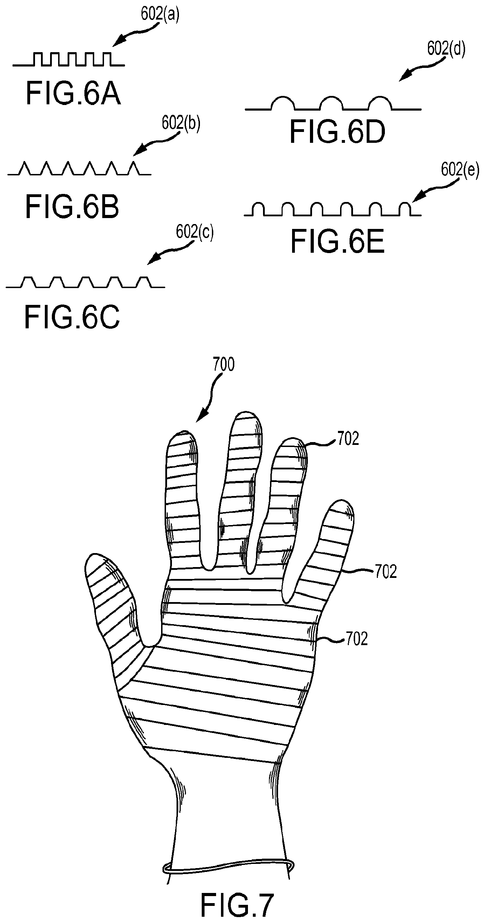

A glove according to the exemplary embodiments of the invention can additionally or alternatively have a pattern formed therein at various positions that allows easier flexing of the glove material. Some exemplary patterns are shown in Figures 6-6E and each provides additional material for opening and/or closing the hand to reduce biasing forces when stretched, and reduce or eliminate a baggy fit when not stretched. The pattern may be one or more of the following:
(1) Any feature with about 10 mm or less of total height or depth that permits a glove to flex with less force when the hand is opened, closed or both.
(2) The material forming the glove may be ribbed, wherein the ribs (which are folds of material) include excess material to permit expansion of the glove and the ribs may extend about 0.3175cm (1/8") or less beyond the outer surface of the glove on either the dorsum side, palm side, between the fingers, between the thumb and fingers, or some combination thereof.
   Figure 2 illustrates a dorsum D side of hand 200 and a palm side P of hand 200.
(3) The material forming the glove can have a pattern of (e.g., alternating) raised portions and/or depressions, wherein the center of each raised portion or depression is preferably no greater than 0,635 cm (1/4" inches) apart from other portions, and most preferably no greater than 0.3175cm (1/8") to 0.079 cm (1/32") apart. The raised or depressed portions are preferably formed in one or more of cross-sectional shapes from the group consisting of: semioval, semi-circle, square, rectangular, triangular, three-sided pyramidal
   and four-sided pyramidal. The pattern may follow the natural contour of the hand where desired. This can create nonlinear and asymmetrical patterns.

A glove can include a more concentrated pattern of patterns proximate areas corresponding to areas of the digits and hand that bend or stretch, thereby allowing easier bending at the areas that need to bend more, such as at one or more of the MCP, PIP, DIP, or IP joints, or in area 32 between the thumb and index finger, or in the web spaces between the other fingers. As another example, a pattern may be present and/or more concentrated along the axis of the dorsum of the little finger, or be more concentrated from the mid hand to the mid proximal phalanx. Or, the pattern may cover any portion of the dorsum or palm. On the index finger, the pattern may cover the distal hand to the proximal phalanx. The pattern may be biased toward the ulnar side of the hand where biasing forces are greatest. On the thumb MCP joint, the pattern may predominate on the dorsum, but might extend into area 32. Additionally, the pattern may be placed along the longitudinal axis of one or more of the fingers and/or thumb.

A glove including a pattern might include the same pattern or different patterns, such as one pattern at one or more of the joints, and another between the spaces between the fingers. For example, alternating raised portions or depressions in the form of a four-sided pyramid may be at any suitable location on the gloves, including on part of or the entire palm side of the glove, and there may be ribs running into the spaces between the fingers, or on the flexor surface of the gloves to relieve biasing forces of extending fingers associated with pre-relaxed surgical gloves. The ribs allow easier expansion to decrease the loads on the digits with abduction of the fingers or wide stretching of the hand, in the same fashion as one would need to play far apart piano keys. The glove can include a pattern over or under any of the joints of one or more of the digits. Further, a pattern can be placed in the mid palm to provide some relaxation during maximal extension of the fingers.

### Other Exemplary Embodiments

As shown in Figures 28A-28C, a glove according to the invention has patterns formed as ribs. In this embodiment ribs 1000 are positioned at the top of each MCP joint and either encircle, or are on the top and palm side, of each PIP joint and DIP joint. The ribs also encircle the thumb PIP and IP joint, and extend along the palm and into the space between the thumb and index finger. There are four separate, non-connected ribs positioned at the thumb PIP joint; four separate, non-connected ribs positioned at the index finger MCP joint, five separate, non-connected ribs at the MCP joint of the middle finger; six separate, non-connected ribs at the MCP joint of the ring finger; and seven separate, non-connected ribs at the MCP joint of the little finger.

In this embodiment, one rib 1000, which is the fourth rib (as measured from the distal ends of the fingers) for each of the index finger, middle finger, ring finger and little finger, is continuous to assist in flexion of the hand between the fingers. Another rib 1000, which is the fifth rib (as measured form the distal ends of the fingers) for each of the middle finger, ring finger and little finger, is continuous to assist in flexing of the hand. Another rib 1000 is the sixth rib (as measured from the distal ends of the fingers) for each of the ring finger and little finger and assists in flexing of the hand. The IP joint of the thumb has four separate, non-connected ribs 1002, and the PIP and DIP joint of each finger has three separate, non-connected ribs 1004.

The ribs in this example have a curved relief at the MCP joints, wherein the end of each rib at an MCP joint is preferably between 1mm to 3mm closer to the distal end of the respective finger on which it is positioned than the center of the rib.

There are also three separate, non-connected ribs 1006 in the palm section, wherein the uppermost rib extends under the little finger, the center rib extends to approximately a position between the little finger and ring finger, and the lower rib extends to approximately the ring finger. There is also an additional rib 1008 running along the muscle of the thumb to assist in flexing the thumb towards the palm or fingers.

Additionally, this example has ribs 1010 extending longitudinally between the MCP joint and PIP joint of each finger, the PIP joint and DIP joint of each finger, the PIP joint and the IP joint of the thumb, and distal of the IP joint of the thumb. Each of these ribs can expand by 1mm to 10mm.

Patterns according to the invention may provide between 1mm to 250mm of extra material depending upon their location on the glove. For example, a pattern positioned at the MCP joint of the: (a) index finger may provide 2mm to 15mm, or 2mm to 11mm of extra material, (b) the middle finger may provide 3mm to 20mm, or 3mm to 15mm, of extra material, (c) the ring finger may include 4mm to 25mm, or 4mm to 24mm, of extra material, and (d) the little finger may include 5mm to 30mm, or 5mm to 35mm, of extra material. A pattern positioned at the PIP and/or DIP joint of the: (a) index finger may provide 1mm to 8mm, or 1mm to 10mm, of extra material, (b) middle finger may provide 1mm to 11mm, or 2mm to 10mm, of extra material, and (c) ring finger may include 2mm to 13mm, or 2mm to 10mm, of extra material. A pattern positioned at the thumb PIP joint may provide for 5mm to 20mm of extra material, and a pattern positioned at the thumb IP joint may provide for 8mm to 20mm of extra material.

A pattern positioned between the thumb and index finger may provide 20mm to 250mm of extra material and pattern positioned between any two fingers may provide 20mm to 200mm of extra material. A pattern on the palm may provide 30mm to 350mm of extra material.

Some non-limiting examples of gloves having different patterns are illustrated in Figures 6-11. Figure 6 illustrates a glove 600 having a pattern 602. As illustrated, pattern 602 covers all of the joints of the digits, the space between the thumb and index finger, and the spaces between each finger. Pattern 602, or any suitable pattern used with the gloves described herein, could cover any suitable portion of the glove, such as one or more of: the portion corresponding to one or more joints, the portion corresponding to the space between the thumb and index finger, any or all spaces between the fingers, the dorsum, and the palm.

Figures 6A-6E illustrate close up, side views of exemplary structures suitable for pattern 602. Figure 6A illustrates pattern 602(a) as raised square or rectangular sections. Figure 6B illustrates pattern 602(b) as raised triangular sections, which would be pyramidal if viewed in three dimensions. Figure 6C illustrates pattern 602(2) as raised flat-topped pyramidal sections. Figure 6D illustrates pattern 602(d) as raised broad partial ovoid or partial spherical sections. Figure 6E illustrates pattern 602(e) as raised dome-like sections. Any suitable structures, or combination of structures, however, could be used to form pattern 602.

Figure 7 illustrates a glove 700 according to various embodiments of the invention with ribs 702 to assist with easier flexing. Exemplary ribs 702 are formed in the material of glove 700 and are preferably no higher than about 2 mm, although any suitable height can be used. The spacing between the ribs may be any suitable spacing and may vary at different areas of the glove. Preferably, the ribs 702 are spaced apart between 0.159 cm (1/16") and
0.635 cm (1/4").

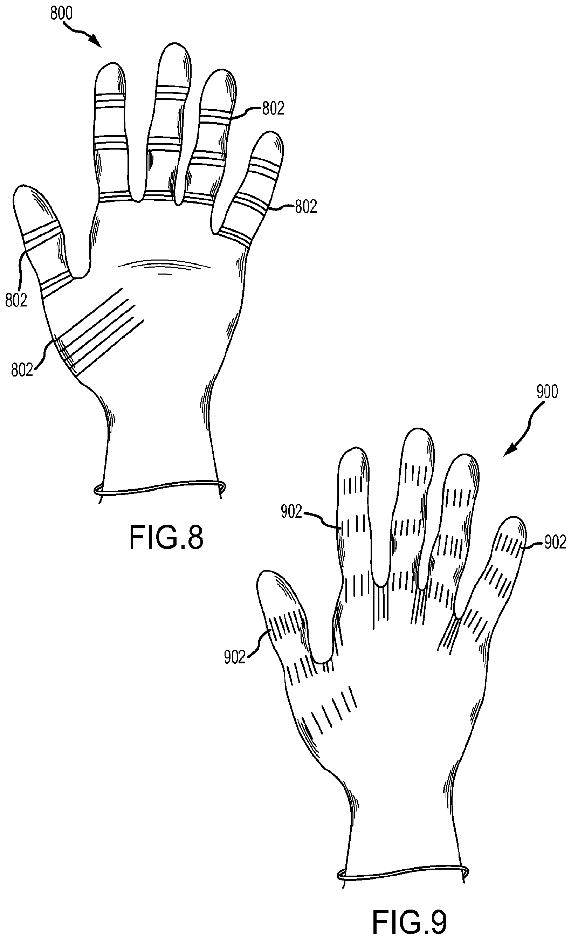

Figure 8 illustrates a glove 800 according to further exemplary embodiments of the invention with ribs 802 to assist with easier flexing. Exemplary ribs 802 can be the same as previously described ribs 702, except ribs 802 only extend along the portions of the glove that correspond to the locations of the joints.

Figure 9 illustrates a glove 900 according to yet further exemplary embodiments of the invention with ribs 902 to assist with easier flexing. Exemplary ribs 902 are formed in the material of glove 900, are axially-aligned along the digits, and are preferably no higher than 2mm, although any suitable height can be used. The spacing between the ribs may be any suitable spacing and may vary at different areas of the glove. Preferably, the ribs 902 are spaced apart between 0.159 cm (1/16") and 0.635 cm (1/4").

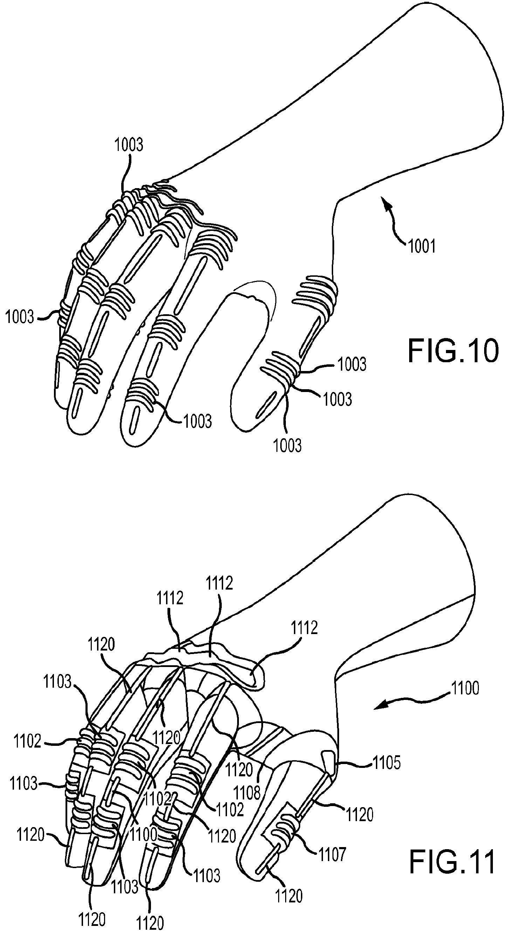

Figure 10 illustrates a glove 1001 according to additional exemplary embodiments of the invention with ribs 1003 to assist with easier flexing. Ribs 1002 are formed in the material of glove 1001 and are preferably no higher than 2mm, although any suitable height can be used. The spacing between the ribs 1003 may be any suitable spacing and may vary at different areas of the glove. Preferably, the ribs 1003 are spaced apart between 0.159 cm (1/16") and 0.635 cm (1/4")

Figure 11 illustrates another glove 1100 according to various embodiments of the invention. Glove 1100 includes alternating patterned / textured seetions that correspond with the location of each joint separated by non- patterned / non-textured sections that are ribs to provide extra material when the hand or a finger flexes.

These folds of material (ribs) can be located in any suitable place, including between any two joints, between any fingers, between the thumb and index finger, on the dorsum of the hand and/or on the palm of the hand. The folds can provide any suitable amount of material to permit expansion, and in one embodiment provide between 0.159 cm (1/16") and 0.635 cm (1/4") of extra material. The folds preferably extend outward away from the hand and/or fingers. In the illustrated example, glove 1100 includes a flexible section 1112 corresponding to the MCP of each finger, a flexible section 1102 corresponding to the PIP of each finger, and a flexible section 1103 corresponding to the DIP of each finger. There is a flexible section 1105 corresponding to the MCP of the thumb and a flexible section 1107 corresponding to the IP of the thumb. There is also a longitudinally oriented pattern 1120 between each of the flexible sections and distal from the DIP joints and IP joint that allows for circumferential expansion of the gloves to minimize biasing forces associated with natural digital circumferential expansion that occurs with normal digital flexion.

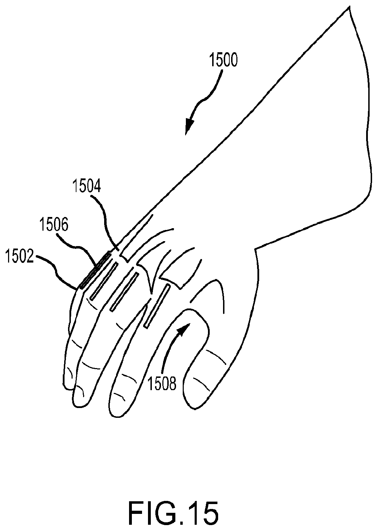

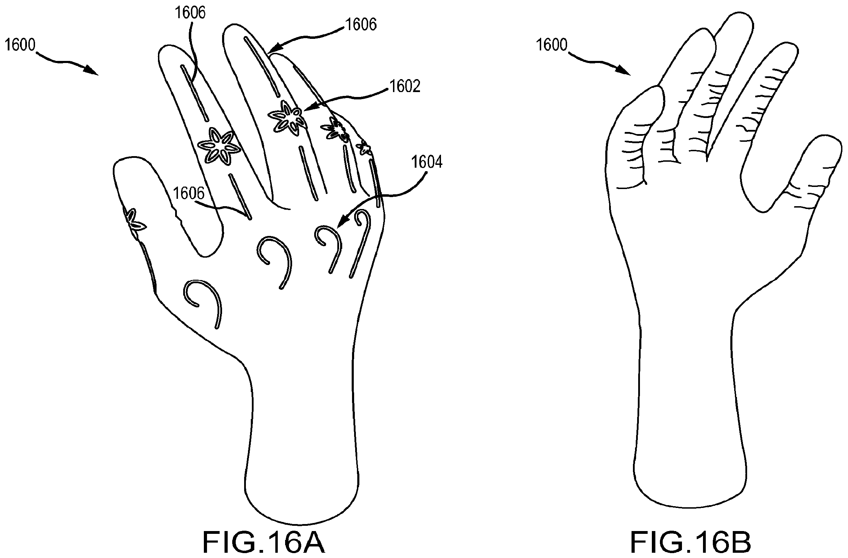

Figures 15 and 16(a) and (b) illustrate additional exemplary gloves 1500 and 1600, respectively. Glove 1500 includes PIP joint modifiers 1502, MCP expansion ridges 1504, a proximal expansion groove 1506, and palm expansion channels 1508 in accordance with exemplary embodiments of the invention.

Glove 1600 includes joint strain relief elements 1602, 1604 and longitudinal expansion relief ridges 1606. Strain relief elements 1602, 1604 can be of any suitable shape that provides a desired amount of stain relief-e.g., greater than or equal to 5%, 10%, 25%, 35%, 50%, or the like. As illustrated, elements 1602, 1604 can be decorative, as long as they provide the desired relief of biasing forces. Similarly, ridges 1606 can provide a desired amount of expansion relief, such as greater than or equal to 5%, 10%, 25%, 35%, 50%, or the like.

As previously mentioned, it is also preferred that a glove according to the invention be able to compensate for the expansion of the fingers' respective circumferences when flexed. Figure 22 illustrates a relationship between the circumferences of fingers when straight and when flexed. A digit circumference can increase from about 15% to about 25% from an extended position to a flex position.

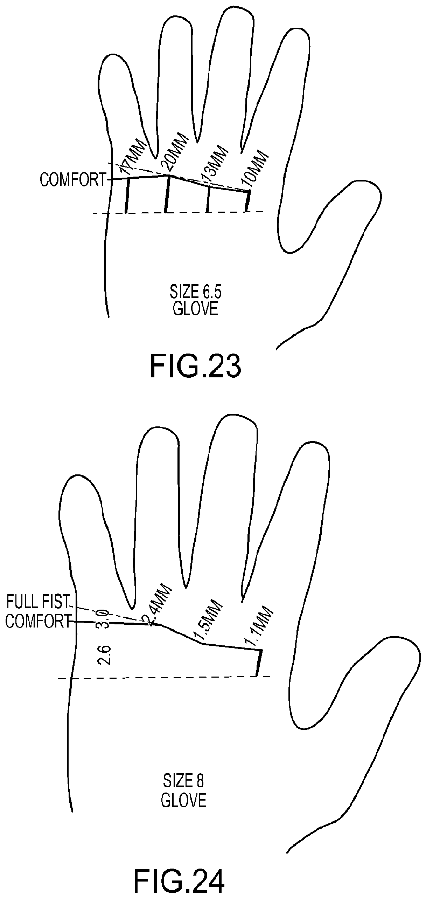

Figures 23-24 illustrate an amount of stretch and a location for desired stretch in accordance with specific exemplary embodiments of the disclosure. In the illustrated examples, stretch for a size 6.5 glove corresponding to the MCP joints in a hand is illustrated on Figure 23. Similarly in figure 24, an amount of stretch in mm is illustrated for a size 8 glove.

### Materials

A glove according to various aspects of exemplary embodiments of the invention can additionally or alternatively include one or both of the following:

The material forming the glove may have varying thicknesses such that thinner portions are used at positions corresponding to one or more joints on a hand (e.g., where joints or the hand are flexed). For example, a thickness can be reduced by 5%, 10%, 25%, 35%, or 50% in any portion of the glove that must flex when the hand and/or one of the digits is opened or closed. For example, the thickness of the material in one or more joint areas or a dorsum region, or at the folds in the palm, or between fingers, or between the thumb and the index finger may be reduced as compared to a thickness of the glove material at other locations.

A glove may be comprised of multiple materials wherein a more flexible material (also called a first material) is used at positions corresponding to joints on a hand (e.g., where the digit joints or the hand are flexed). A more puncture-resistant material (also called a second material) may be used at other locations. For example, an elongation at break of the more puncture-resistant material can be about 5%, 10%, 25%, 35%, 50%, 100%, 200% or 300% less than the elongation at break of the more flexible material. The more puncture resistant second material can be 5%, 10%, 25%, 35%, 50%, 100%, 200%, or 300% more puncture resistant, e.g., as determined according to ASTM F1342. The second material may be positioned at one or more finger tips, tip of the thumb, or at any suitable location that permits the hand to open and close without much resistance.

A glove according to various aspects of exemplary embodiments of the invention can additionally or alternatively include one or both of the following:

The material forming the glove may have varying thicknesses such that thinner portions are used at positions corresponding to one or more joints on a hand (e.g., where joints or the hand are flexed). For example, a thickness can be reduced by 5%, 10%, 25%, 35%, or 50% in any portion of the glove that must flex when the hand and/or one of the digits is opened or closed. For example, the thickness of the material in one or more joint areas or a dorsum region, or at the folds in the palm, or between fingers, or between the thumb and the index finger may be reduced as compared to a thickness of the glove material at other locations.

A glove may be comprised of multiple materials wherein a more flexible material (also called a first material) is used at positions corresponding to joints on a hand (e.g., where the digit joints or the hand are flexed). A more puncture-resistant material (also called a second material) may be used at other locations. For example, an elongation at break of the more puncture-resistant material can be about 5%, 10%, 25%, 35%, 50%, 100%, 200% or 300% less than the elongation at break of the more flexible material. The more puncture resistant second material can be 5%, 10%, 25%, 35%, 50%, 100%, 200%, or 300% more puncture resistant, e.g., as determined according to ASTM F1342. The second material may be positioned at one or more finger tips, tip of the thumb, or at any suitable location that permits the hand to open and close without much resistance.

A glove according to aspects of the invention, in addition to including all of the other features of claim 1, may include portions where the glove material itself is thinner so that less force is required to stretch the thinner portion. Such a glove may also have portions of material thinner than a standard glove would have in order to be more resistant to being punctured.

A glove according to aspects of the invention, in addition to including all of the other features of claim 1, may also be comprised of multiple materials, wherein one material is more flexible and is positioned at locations wherein easier flexing of the gloves is desired.

An aspect of the invention also includes the concept of placing one glove according to the present invention (an outer glove) over another (an inner glove) to create a double glove. A double glove could be packaged as one item in a single package thereby eliminating the packaging of the second glove.

The outer glove may be slightly larger than the inner glove in order to facilitate easier placement of one over the other. For instance a size 8 double might include a size 8 inner glove and a size 8.1 outer glove. Additionally, the inner glove may have a micro-texturing on the outside surface and/or the outer glove may have a micro-texturing on the inner surface to facilitate easier placement of one over the other.

Having thus described preferred embodiments of the invention, other variations and embodiments will become apparent to those skilled in the art. The scope of the present invention is thus not limited to any particular embodiment, but is instead set forth in the appended claims.

## Claims

1. A surgical glove comprising:
one or more patterns (1003) to provide an additional length of material along a dorsal surface of at least one finger of the surgical glove, **characterised by**
at least one longitudinal rib (1010) extending between two joints of a finger or two joints of the thumb, wherein the longitudinal rib (1010) provides between 1mm and 10mm of additional material.

2. The surgical glove of claim 1 wherein the one or more patterns is a plurality of separate, non-connected ribs.

3. The surgical glove of claim 1 or claim 2, wherein at least one of the one or more patterns is located at the MCP joint portion of an index finger of the surgical glove and provides 2 mm to 15 mm of additional length of material.

4. The surgical glove of claim 3, wherein the one or more patterns also comprises a pattern corresponding to an area proximate an MCP joint portion of a middle finger of the surgical glove and provides 3 mm to 20 mm of additional length of material.

5. The surgical glove of claim 4, wherein the one or more patterns also comprises a pattern corresponding to an area proximate an MCP joint portion of a ring finger of the surgical glove and provides 4 mm to 25 mm of additional length of material.

6. The surgical glove of claim 5, wherein the one or more patterns also comprises a pattern corresponding to an area proximate an MCP joint portion of a little finger of the surgical glove and provides 5 mm to 35 mm of additional length of material.

7. The surgical glove of claim 1, wherein the one or more patterns comprises at least one of (a) a pattern corresponding to an area proximate a PIP joint portion of an index finger of the surgical glove, and (b) a pattern corresponding to an area proximate a DIP joint portion of the index finger of the surgical glove, and provides 1 mm to 10 mm of additional length of material at the PIP joint portion and also at the DIP joint portion.

8. The surgical glove of claim 1, wherein the one or more patterns comprises at least one of (a) a pattern corresponding to an area proximate a PIP joint portion of a middle finger of the surgical glove, and (b) a pattern corresponding to an area proximate a DIP joint portion of a middle finger of the surgical glove, and provides 1 mm to 11 mm of additional length of material at the PIP joint portion and also at the DIP joint portion.

9. The surgical glove of claim 1, wherein the one or more patterns comprises at least one of (a) a pattern corresponding to an area proximate a PIP joint portion of a ring finger, and (b) a pattern corresponding to an area proximate a DIP joint portion of a ring finger of the surgical glove, and provides 2 mm to 13 mm of additional length of material at the PIP joint portion and also at the DIP joint portion.

10. The surgical glove of claim 1, wherein the one or more patterns comprises a pattern corresponding to an area proximate an MCP joint, wherein the pattern provides about one-half of the additional length of material at the MCP joint and about one-half of the additional material is at other portions of the finger.

11. The surgical glove of claim 1, wherein the surgical glove comprises a plurality of longitudinal ribs (1010), wherein each longitudinal rib (1010) extends between two joints on a finger or the thumb, and each longitudinal rib provides between 1mm and 3mm of additional material.

12. The surgical glove of claim 1 that is formed to provide an additional 8mm to 15mm of material along the length of the top surface of the index finger when the hand is in the position of a fist, as compared to the length of the top surface of the index finger when in its extended position and straight.

13. The surgical glove of claim 1 that is formed to provide an additional 10mm to - 18mm of material along the length of the top surface of the middle finger when the hand is in the position of a fist, as compared to the length of the top surface of the middle finger when in its extended position and straight.

14. The surgical glove of claim 1 that is formed to provide an additional 15mm to - 30mm of material along the length of the top surface of the ring finger when the hand is in the position of a fist, as compared to the length of the top surface of the ring finger when in its extended position and straight.

15. The surgical glove of claim 1 that is formed to provide an additional 20mm to - 40mm of material along the length of the top surface of the little finger when the hand is in the position of a fist, as compared to the length of the top surface of the little finger when in its extended position and straight.

16. The surgical glove of claim 1, wherein the one or more patterns comprises a pattern corresponding to an area proximate each MCP joint, wherein the pattern provides about one-half of the additional length of material for each finger at the MCP joint and about one-half of the additional material for each finger is at other portions of the finger.

## Patentansprüche

1. Operationshandschuh, welcher umfasst:
Ein oder mehrere Muster (1003), die dazu dienen, längs einer dorsalen Fläche von mindestens einem Finger des Operationshandschuhs zusätzliche Materiallänge zu bieten, **dadurch gekennzeichnet, dass** zwischen zwei Gelenken eines Fingers oder zwei Gelenken des Daumens sich mindestens eine Längsrippe (1010) erstreckt, wobei diese Längsrippe (1010) zwischen 1 mm und 10 mm an zusätzlichem Material bereitstellt.

2. Operationshandschuh nach Anspruch 1, bei welchem das eine oder die mehreren Muster durch eine gewisse Anzahl von getrennten, nicht miteinander verbundenen Rippen darstellt wird.

3. Operationshandschuh nach Anspruch 1 oder Anspruch 2, bei welchem mindestens eines des einen Musters oder der mehreren Muster sich am Mittelhandfinger-Gelenkabschnitt eines Zeigefingers des Operationshandschuhs befindet und 2 mm bis 15 mm an zusätzlicher Materiallänge bereitstellt.

4. Operationshandschuh nach Anspruch 3, bei welchem das eine oder die mehreren Muster auch ein Muster umfassen, welches einer Fläche nahe einem Mittelhandfinger-Gelenkabschnitt eines Mittelfingers des Operationshandschuhs entspricht und 3 mm bis 20 mm an zusätzlicher Materiallänge bereitstellt.

5. Operationshandschuh nach Anspruch 4, bei welchem das eine oder die mehreren Muster auch ein Muster umfassen, welches einer Fläche nahe einem Mittelhandfinger-Gelenkabschnitt eines Ringfingers des Operationshandschuhs entspricht und 4 mm bis 25 mm an zusätzlicher Materiallänge bereitstellt.

6. Operationshandschuh nach Anspruch 5, bei welchem das eine oder die mehreren Muster auch ein Muster umfassen, welches einer Fläche nahe einem Mittelhandfinger-Gelenkabschnitt eines kleinen Fingers des Operationshandschuhs entspricht und 5 mm bis 35 mm an zusätzlicher Materiallänge bereitstellt.

7. Operationshandschuh nach Anspruch 1, bei welchem das eine oder die mehreren Muster mindestens (a) ein Muster, welches einer Fläche nahe einem Fingergrundglied-Gelenkabschnitt eines Zeigefingers des Operationshandschuhs entspricht, und (b) ein Muster, welches einer Fläche nahe einem Fingerendglied-Gelenkabschnitt des Zeigefingers des Operationshandschuhs entspricht, umfasst und 1 mm bis 10 mm an zusätzlicher Materiallänge am Fingergrundglied-Gelenkabschnitt und auch am Fingerendglied-Gelenkabschnitt bereitstellt.

8. Operationshandschuh nach Anspruch 1, bei welchem das eine oder die mehreren Muster mindestens (a) ein Muster, welches einer Fläche nahe einem Fingergrundglied-Gelenkabschnitt eines Mittelfingers des Operationshandschuhs entspricht, und (b) ein Muster, welches einer Fläche nahe einem Fingerendglied-Gelenkabschnitts des Mittelfingers des Operationshandschuhs entspricht, umfasst und 1 mm bis 11 mm an zusätzlicher Materiallänge am Fingergrundglied-Gelenkabschnitt und auch am Fingerendglied-Gelenkabschnitt bereitstellt.

9. Operationshandschuh nach Anspruch 1, bei welchem das eine oder die mehreren Muster mindestens (a) ein Muster, welches einer Fläche nahe einem Fingergrundglied-Gelenkabschnitt eines Ringfingers des Operationshandschuhs entspricht, und (b) ein Muster, welches einer Fläche nahe einem Fingerendglied-Gelenkabschnitt des Ringfingers des Operationshandschuhs entspricht, umfasst und 2 mm bis 13 mm an zusätzlicher Materiallänge am Fingergrundglied-Gelenkabschnitt und auch am Fingerendglied-Gelenkabschnitt bereitstellt.

10. Operationshandschuh nach Anspruch 1, bei welchem das eine oder die mehreren Muster ein Muster umfassen, welches einer Fläche nahe einem Mittelhandfinger-Gelenk entspricht, wobei dieses Muster ungefähr eine Hälfte der zusätzlichen Materiallänge am Mittelhandfinger-Gelenk bereitstellt und ungefähr eine Hälfte des zusätzlichen Materials sich an anderen Abschnitten des Fingers befindet.

11. Operationshandschuh nach Anspruch 1, bei welchem der Operationshandschuh eine gewisse Anzahl von Längsrippen (1010) umfasst, wobei jede Längsrippe (1010) sich zwischen zwei Gelenken eines Fingers oder des Daumens erstreckt und jede Längsrippe zwischen 1 und 3 mm an zusätzlichem Material bereitstellt.

12. Operationshandschuh nach Anspruch 1, welcher dergestalt ausgebildet ist, dass er zusätzliche 8 mm bis 15 mm an Material längs der Länge der Oberseite des Zeigefingers bereitstellt, wenn die Hand sich in der Fauststellung befindet im Vergleich mit der Länge der Oberseite des Zeigefingers, wenn sie sich in der ausgestreckten Stellung befindet und gerade ist.

13. Operationshandschuh nach Anspruch 1, welcher dergestalt ausgebildet ist, dass er zusätzliche 10 mm bis 18 mm an Material längs der Länge der Oberseite des Mittelfingers bereitstellt, wenn die Hand sich in der Fauststellung befindet im Vergleich mit der Länge der Oberseite des Mittelfingers, wenn sie sich in der ausgestreckten Stellung befindet und gerade ist.

14. Operationshandschuh nach Anspruch 1, welcher dergestalt ausgebildet ist, dass er zusätzliche 15 mm bis 30 mm an Material längs der Länge der Oberseite des Ringfingers bereitstellt, wenn die Hand sich in der Fauststellung befindet im Vergleich mit der Länge der Oberseite des Ringfingers, wenn sie sich in der ausgestreckten Stellung befindet und gerade ist.

15. Operationshandschuh nach Anspruch 1, welcher dergestalt ausgebildet ist, dass er zusätzliche 20 mm bis 40 mm an Material längs der Länge der Oberseite des kleinen Fingers bereitstellt, wenn die Hand sich in der Fauststellung befindet im Vergleich mit der Länge der Oberseite des kleinen Fingers, wenn sie sich in der ausgestreckten Stellung befindet und gerade ist.

16. Operationshandschuh nach Anspruch 1, bei welchem das eine oder die mehreren Muster ein Muster umfassen, welches einer Fläche nahe einem jeden Mittelhandfingergelenk entspricht, wobei dieses Musters ungefähr eine Hälfte der zusätzlichen Materiallänge für jeden Finger am Mittelhandfingergelenk bereitstellt und ungefähr eine Hälfte des zusätzlichen Materials für jeden Finger sich an anderen Abschnitten des Fingers befindet.

## Revendications

1. Gant chirurgical comprenant :
une ou plusieurs configurations (1003) pour fournir une longueur supplémentaire de matériau le long d'une surface dorsale d'au moins un doigt du gant chirurgical,
**caractérisé par**
au moins une nervure longitudinale (1010) s'étendant entre deux articulations d'un doigt ou deux articulations du pouce, où la nervure longitudinale (1010) fournit entre 1 mm et 10 mm de matériau supplémentaire.

2. Gant chirurgical selon la revendication 1 où l'une ou plusieurs configurations est une pluralité de nervures séparées, non connectées.

3. Gant chirurgical selon la revendication 1 ou la revendication 2, où au moins une de l'une ou plusieurs configurations est située au niveau de la partie d'articulation MCP d'un index du gant chirurgical et fournit de 2 mm à 15 mm de longueur supplémentaire de matériau.

4. Gant chirurgical selon la revendication 3, où l'une ou plusieurs configurations comprend également une configuration correspondant à une zone à proximité d'une partie d'articulation MCP d'un majeur du gant chirurgical et fournit de 3 mm à 20 mm de longueur supplémentaire de matériau.

5. Gant chirurgical selon la revendication 4, où l'une ou plusieurs configurations comprend également une configuration correspondant à une zone à proximité d'une partie d'articulation MCP d'un annulaire du gant chirurgical et fournit de 4 mm à 25 mm de longueur supplémentaire de matériau.

6. Gant chirurgical selon la revendication 5, où l'une ou plusieurs configurations comprend également une configuration correspondant à une zone à proximité d'une partie d'articulation MCP d'un auriculaire du gant chirurgical et fournit de 5 mm à 35 mm de longueur supplémentaire de matériau.

7. Gant chirurgical selon la revendication 1, où l'une ou plusieurs configurations comprend au moins une (a) d'une configuration correspondant à une zone à proximité d'une partie d'articulation PIP d'un index du gant chirurgical, et (b) d'une configuration correspondant à une zone à proximité d'une partie d'articulation DIP de l'index du gant chirurgical et fournit de 1 mm à 10 mm de longueur supplémentaire de matériau au niveau de la partie d'articulation PIP et également au niveau de la partie d'articulation DIP.

8. Gant chirurgical selon la revendication 1, où l'une ou plusieurs configurations comprend au moins une (a) d'une configuration correspondant à une zone à proximité d'une partie d'articulation PIP d'un majeur du gant chirurgical, et (b) d'une configuration correspondant à une zone à proximité d'une partie d'articulation DIP d'un majeur du gant chirurgical et fournit de 1 mm à 11 mm de longueur supplémentaire de matériau au niveau de la partie d'articulation PIP et également au niveau de la partie d'articulation DIP.

9. Gant chirurgical selon la revendication 1, où l'une ou plusieurs configurations comprend au moins une (a) d'une configuration correspondant à une zone à proximité d'une partie d'articulation PIP d'un annulaire, et (b) d'une configuration correspondant à une zone à proximité d'une partie d'articulation DIP d'un annulaire du gant chirurgical et fournit de 2 mm à 13 mm de longueur supplémentaire de matériau au niveau de la partie d'articulation PIP et également au niveau de la partie d'articulation DIP.

10. Gant chirurgical selon la revendication 1, où l'une ou plusieurs configurations comprend une configuration correspondant à une zone à proximité d'une articulation MCP, où la configuration fournit environ moitié de la longueur supplémentaire de matériau au niveau de l'articulation MCP et environ moitié du matériau supplémentaire est au niveau d'autres parties du doigt.

11. Gant chirurgical selon la revendication 1, où le gant chirurgical comprend une pluralité de nervures longitudinales (1010), où chaque nervure longitudinale (1010) s'étend entre deux articulations sur un doigt ou le pouce, et chaque nervure longitudinale fournit entre 1 mm et 3 mm de matériau supplémentaire.

12. Gant chirurgical selon la revendication 1 qui est formé pour fournir de 8 mm à 15 mm supplémentaires de matériau le long de la longueur de la surface supérieure de l'index quand la main est dans la position d'un poing, par rapport à la longueur de la surface supérieure de l'index quand il est dans sa position étendue et droite.

13. Gant chirurgical selon la revendication 1 qui est formé pour fournir de 10 mm à 18 mm supplémentaires de matériau le long de la longueur de la surface supérieure du majeur quand la main est dans la position d'un poing, par rapport à la longueur de la surface supérieure du majeur quand il est dans sa position étendue et droite.

14. Gant chirurgical selon la revendication 1 qui est formé pour fournir de 15 mm à 30 mm supplémentaires de matériau le long de la longueur de la surface supérieure de l'annulaire quand la main est dans la position d'un poing, par rapport à la longueur de la surface supérieure de l'annulaire quand il est dans sa position étendue et droite.

15. Gant chirurgical selon la revendication 1 qui est formé pour fournir de 20 mm à 40 mm supplémentaires de matériau le long de la longueur de la surface supérieure de l'auriculaire quand la main est dans la position d'un poing, par rapport à la longueur de la surface supérieure de l'auriculaire quand il est dans sa position étendue et droite.

16. Gant chirurgical selon la revendication 1, où l'une ou plusieurs configurations comprend une configuration correspondant à une zone à proximité de chaque articulation MCP, où la configuration fournit environ moitié de la longueur supplémentaire de matériau pour chaque doigt au niveau de l'articulation MCP et environ moitié du matériau supplémentaire pour chaque doigt est au niveau d'autres positions du doigt.
